# EUROPEAN PATENT SPECIFICATION

(11) **EP 3 427 775 B1**
(45) Date of publication and mention of the grant of the patent: **24.05.2023**
(21) Application number: 17763419.3
(22) Date of filing: 10.03.2017
(51) Int. Cl.: A61M 39/26, A61M 25/00

(54) **MEDICINE INJECTION CATHETER AND MEDICINE INJECTION SYSTEM**
MEDIKAMENTENINJEKTIONSKATHETER UND MEDIKAMENTENINJEKTIONSSYSTEM
CATHÉTER D'INJECTION DE MÉDICAMENT ET SYSTÈME D'INJECTION DE MÉDICAMENT

(30) Priority: 11.03.2016 JP 2016047972
(43) Date of publication of application: 16.01.2019
(73) Proprietor: Terumo Kabushiki Kaisha, Tokyo 151-0072 (JP)
(72) Inventor: SHIMADA Naoya, Ashigarakami-gun Kanagawa 259-0151 (JP); ISHII Naoki, Ashigarakami-gun Kanagawa 259-0151 (JP)
(74) Representative: Prüfer & Partner mbB Patentanwälte · Rechtsanwälte
(86) International application number: PCT/JP2017/009662
(87) International publication number: WO 2017/155079

(56) References cited:
- WO-A1-2004/020032
- JP-A- 2009 291 373
- JP-A- 2011 516 205
- US-A- 5 336 222
- US-A- 5 725 524
- US-A1- 2003 195 469

## Description

### Technical Field

The present invention relates to a medication injecting catheter, and more particularly a medication injecting catheter for injecting a medication into a living body through a body wall thereof.

The present invention is also concerned with a medication injecting system that employs such a medication injecting catheter.

### Background Art

When the pumping function of a heart fails due to any of various causes, the heart is unable to pump enough blood required for tissue metabolism in the whole body. As one therapeutic method for such a heart failure, there has been studied a noteworthy process of injecting a medication made of biomaterials such as growth factors, genes, or cells, etc. into a damaged region such as a myocardial infarct.

Such a medication may be injected into the heart by way of a thoracotomy. To make the injecting process less invasive, however, Patent Document 1, for example, discloses a medical instrument having a puncture unit provided on the distal end of a catheter. The puncture unit is used to puncture a body wall such as a cardiac muscle or the like with a needle-like device thereof and to inject the medication through the needle-like device into the body wall The invention is defined in claim 1. Further embodiments of the invention are defined in the dependent claims. Patent document US2003/195469 A1 discloses a medication injection catheter for injecting a medication into a living body.

### Prior Art Document

### Patent Document

Patent Document 1: JP-T-2004-528062

### Summary of Invention

### Technical Problems

However, the disclosed medical instrument is problematic in that the procedure for sticking the needle-like device into the body wall is complex and time-consuming.

Furthermore, since it is not possible to confirm whether the needle-like device has pierced the body wall or not, the medication may possibly leak out of the needle-like device around the body wall when approximately the needle-like device pierces the body wall. If the medication leaks around the body wall, then there is a possibility that it may cause a new disease or it may be difficult to administer a proper amount of medication to the target region.

The present invention has been made in efforts to solve the existing problems described above. It is an object of the present invention to provide a medication injecting catheter which is capable of injecting a medication reliably into a living body from a body wall thereof without leaking the medication in a simple procedure.

It is also an object of the present invention to provide a medication injecting system that employs such a medication injecting catheter.

### Technical Solution

A medication injecting catheter according to the present invention is a medication injecting catheter for injecting a medication into a living body through a body wall thereof, and includes a catheter body having a medication supply lumen defined therein, a tubular body coupled to a distal end of the catheter body and having a lumen defined therein which is held in fluid communication with the medication supply lumen, an injection needle coupled to a distal end of the tubular body, a hollow cylindrical cover member having an inner space defined therein for housing at least the tubular body therein, the cover member being disposed on the distal end of the catheter body for reciprocating movement along axial directions of the catheter body between a first position in which a distal end of the injection needle is housed in the inner space and a second position in which the distal end of the injection needle projects forwardly from the inner space, an opening and closing mechanism having an operating member rotatable in circumferential directions of the catheter body in the inner space in the cover member, for opening and closing the lumen in the tubular body depending on an angular position of the operating member, a motion converting mechanism for converting reciprocating movement of the cover member with respect to the catheter body into rotary movement of the operating member to cause the opening and closing mechanism to close the lumen in the tubular body when the cover member is positioned in the first position and to cause the opening and closing mechanism to open the lumen in the tubular body when the cover member is positioned in the second position, and a resilient member disposed between the catheter body and the cover member for resiliently pressing the cover member forwardly of the catheter body toward the first position.

The tubular member may be pliable, and the opening and closing mechanism may include a diaphragm mechanism for changing the opening area of the lumen in the tubular body depending on the angular position of the operating member. Alternatively, the tubular member may be pliable, and the opening and closing mechanism may include at least one rotor for twisting the tubular body depending on the angular position of the operating member.

The motion converting mechanism should preferably have an operating member guide groove defined helically in an inner circumferential surface of the cover member around a central axis of the cover member, and the operating member should preferably include a projection extending radially of the catheter body in the inner space in the cover member and movably inserted in the operating member guide groove.

Preferably, the cover member should have a partition plate fixedly disposed in the inner space thereof perpendicularly to the axial directions of the catheter body and having a through hole defined therein with the injection needle extending therethrough, and the resilient member should be disposed between a front end of the catheter body and the partition plate of the cover member.

The medication injecting may further include a trap mechanism for trapping a body wall surface of a living body into a front end portion of the cover member by evacuating the inner space in the cover member.

The catheter body and the cover member should preferably have respective contrast markers. The contrast markers make it possible to confirm, from outside, the manner in which the cover member moves and the injection needle pierces a body wall such as a heart muscle or the like. In addition, the practitioner is preferably given information regarding how the catheter body, particularly the distal end thereof, is twisted and the direction in which the catheter body is oriented, depending on how the pattern of the contrast markers appears.

A medication injecting system according to the present invention includes the above medication injecting catheter, a pressure gage for detecting a pressure in the medication supply lumen of the medication injecting catheter, a medication supply for supplying a medication to the medication supply lumen of the medication injecting catheter, and a controller for controlling the supplying of the medication from the medication supply so that the pressure detected by the pressure gage in the medication supply lumen of the medication injecting catheter is constant.

### Advantageous Effects

According to the present invention, the cover member with the inner space defined therein is reciprocally movable between the first position in which the distal end of the injection needle is housed in the inner space and the second position in which the distal end of the injection needle projects forwardly from the inner space, the motion converting mechanism converts reciprocating movement of the cover member with respect to the catheter body into rotary movement of the operating member, and the opening and closing mechanism opens and closes the lumen in the tubular body depending on the angular position of the operating member, to close the lumen in the tubular body when the cover member is positioned in the first position and to open the lumen in the tubular body when the cover member is positioned in the second position. It is thus possible to inject the medication reliably into the living body through the body wall thereof without leaking the medication in a simple procedure.

### Brief Description of Drawings

[FIG. 1]

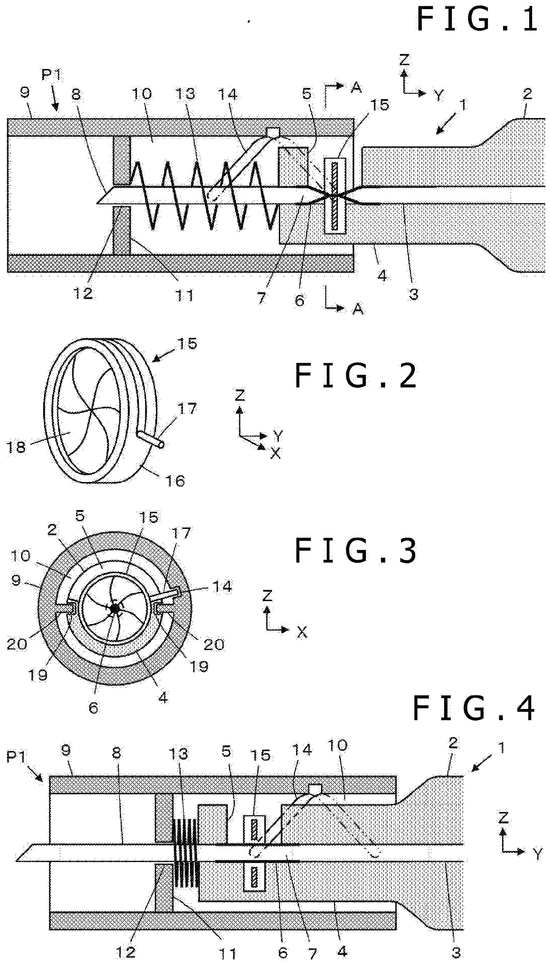

   FIG. 1 is a cross-sectional view of a medication injecting catheter according to Embodiment 1, with a cover member positioned in a first position.
[FIG. 2]
   FIG. 2 is a perspective view of a diaphragm mechanism used in the medication injecting catheter according to Embodiment 1, the diaphragm mechanism being depicted as being closed.
[FIG. 3]
   FIG. 3 is a cross-sectional view taken along line A-A of FIG. 1.
[FIG. 4]
   FIG. 4 is a cross-sectional view of the medication injecting catheter according to Embodiment 1, with the cover member positioned in a second position.
[FIG. 5]

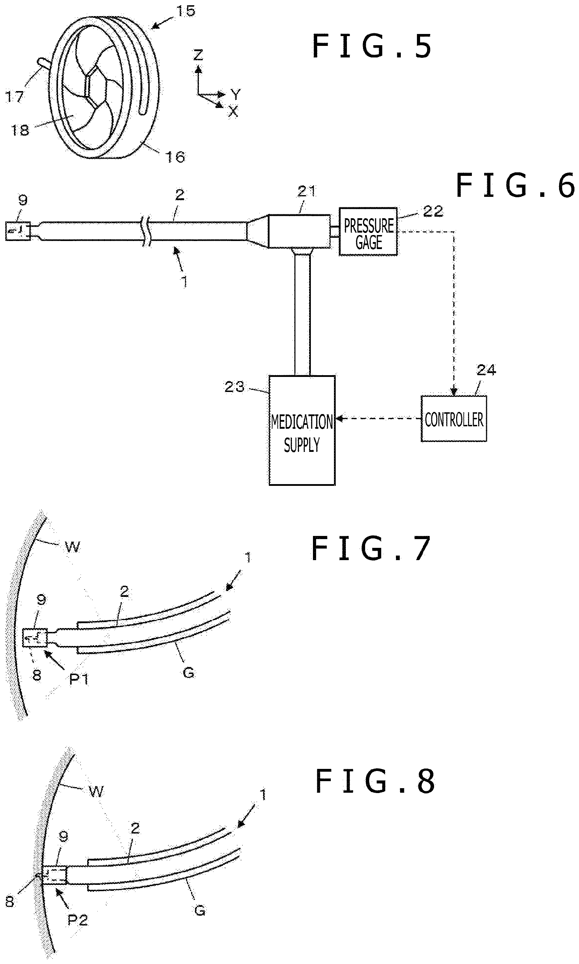

   FIG. 5 is a perspective view of the diaphragm mechanism used in the medication injecting catheter according to Embodiment 1, the diaphragm mechanism being depicted as being open.
[FIG. 6]
   FIG. 6 is a block diagram depicting the makeup of a medical injection system that uses the medication injecting catheter according to Embodiment 1.
[FIG. 7]
   FIG. 7 is a view depicting the medication injecting catheter according to Embodiment 1, with the cover member disposed in the vicinity of the surface of a body wall.
[FIG. 8]
   FIG. 8 is a view depicting the medication injecting catheter according to Embodiment 1, with an injection needle having pierced the body wall.
[FIG. 9]

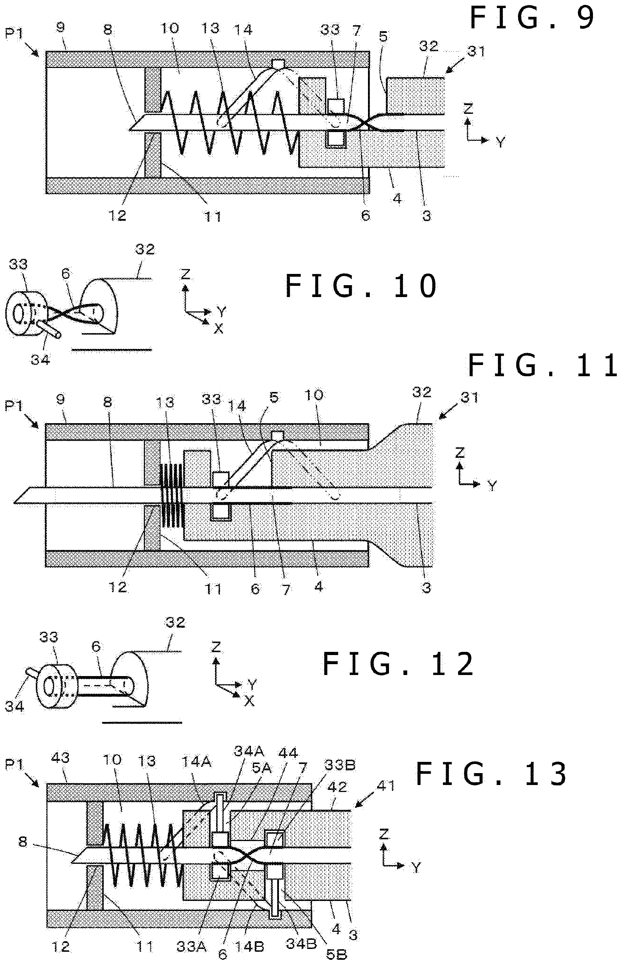

   FIG. 9 is a cross-sectional view of a medication injecting catheter according to Embodiment 2, with a cover member positioned in a first position.
[FIG. 10]
   FIG. 10 is a perspective view of a tubular body of the medication injecting catheter according to Embodiment 2, with the cover member positioned in the first position.
[FIG. 11]
   FIG. 11 is a cross-sectional view of the medication injecting catheter according to Embodiment 2, with the cover member positioned in a second position.
[FIG. 12]
   FIG. 12 is a perspective view of the tubular body of the medication injecting catheter according to Embodiment 2, with the cover member positioned in the second position.
[FIG. 13]
   FIG. 13 is a cross-sectional view of a medication injecting catheter according to Embodiment 3, with a cover member positioned in a first position.
[FIG. 14]

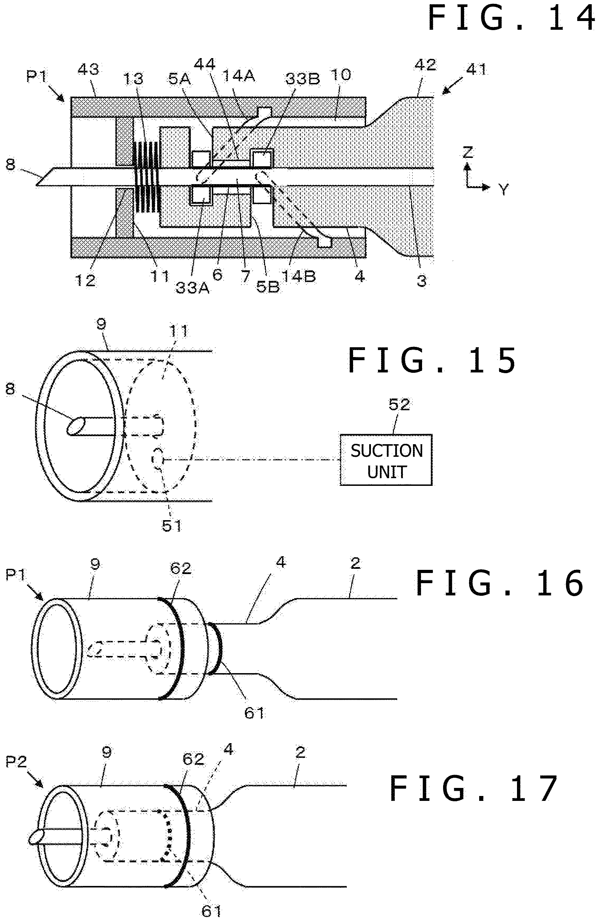

   FIG. 14 is a cross-sectional view of the medication injecting catheter according to Embodiment 3, with the cover member positioned in a second position.
[FIG. 15]
   FIG. 15 is a perspective view of a distal end portion of a medication injecting catheter according to Embodiment 4.
[FIG. 16]
   FIG. 16 is a perspective view of a medication injecting catheter according to Embodiment 5, with a cover member positioned in a first position.
[FIG. 17]
   FIG. 17 is a perspective view of the medication injecting catheter according to Embodiment 5, with the cover member positioned in a second position.

### Modes for Carrying Out the Invention

Embodiments of the present invention will be described below with reference to the accompanying drawings.

### Embodiment 1

FIG. 1 depicts the makeup of a medication injecting catheter 1 according to Embodiment 1. The medication injecting catheter 1 has an elongate catheter body 2 having a medication supply lumen 3 defined therein that extends along an axial direction of the catheter body 2.

The catheter body 2 includes a reduced-diameter portion 4 on its distal end portion, which is smaller in diameter than the rest of the catheter body 2. The reduced-diameter portion 4 has a recess 5 defined therein perpendicularly to the axial direction of the catheter body 2. The recess 5 divides or separates the medication supply lumen 3, with a tubular body 6 disposed across the divided region of the medication supply lumen 3.

The tubular body 6 includes a pliable (compressible) soft tube and has open ends through which a lumen 7 in the tubular body 6 is in fluid communication with the medication supply lumen 3, thereby connecting the divided ends of the medication supply lumen 3. An injection needle 8 is held on the distal end portion of the catheter body 2, and is coupled or connected to the tubular body 6 either directly or through the medication supply lumen 3.

For illustrative purposes, the axial direction of the catheter body 2 will be referred to as a Y direction, the direction in which the recess 5 is defined in the reduced-diameter portion 4 as a +Z direction, and a direction perpendicular to a YZ plane as an X direction.

A hollow cylindrical cover member 9 having a central axis coaxial with the catheter body 2 is disposed on the distal end portion of the catheter body 2 in surrounding relation to the reduced-diameter portion 4. The cover member 9 has an inner space or interior 10 that houses the reduced-diameter portion 4, and may have a partition plate 11 disposed in the inner space in the vicinity of the distal end of the cover member 9 and extending in an XZ plane, dividing the inner space 10 into two areas in the Y direction. The partition plate 11 has a through hole 12 defined centrally in the partition plate 11 and extending in the Y direction. The through hole 12 has a diameter larger than the outside diameter of the injection needle 8, which extends through the through hole 12.

A resilient member 13 which may be in the form of includes a helical spring is disposed between the partition plate 11 in the cover member 9 and the front end of the catheter body 2, and the injection needle 8 extends through the resilient member 13. The resilient member 13 is not limited to a spring, but may be any member that is resilient.

The cover member 9 has an operating member guide groove 14 defined in an inner wall surface of the cover member. The operating member guide groove 14 is disposed only in the inner wall surface of the cover member 9 that lies in a +Z direction, of the entire inner wall surface of the cover member 9, in agreement with the direction along which the recess 5 is defined in the catheter body 2. The operating member guide groove 14 extends helically around the central axis of the cover member 9 along the Y direction.

A diaphragm mechanism (diaphragm) 15 for changing the opening area of the lumen 7 in the tubular body 6 is disposed in the recess 5 in the catheter body 2. The diaphragm mechanism 15 serves as an opening and closing mechanism for changing the opening area of the lumen 7 in the tubular body 6 to open and close the lumen 7 in the tubular body 6 by pressing an outer circumferential portion (outer peripheral surface) of the tubular body 6 all around the tubular body 6 toward the central axis of the tubular body 6. As depicted in FIG. 2, the diaphragm mechanism 15 has a hollow cylindrical frame 16, an operating member 17 extending radially outwardly from the frame 16, and a plurality of blades 18 arrayed circumferentially inside the frame 16. The operating member 17 is movable along an outer circumferential portion of the frame 16 in circumferential directions of the frame 16. The blades 18 are arranged to be opened and closed in an XZ plane depending on the angular position of the operating member 17. The operating member 17 has a distal end positioned in the operating member guide groove 14 defined in the inner wall surface of the cover member 9, so that the angular position of the operating member 17 varies depending on the relative position of the cover member 9 with respect to the catheter body 2 in the Y direction. The operating member guide groove 14 of the cover member 9 serves as a motion converting mechanism.

As depicted in FIG. 3, the reduced-diameter portion 4 of the catheter body 2 has a pair of cover member guide grooves 19 defined in an outer circumferential surface of the reduced-diameter portion 4 of the catheter body 2 and extending in the Y direction at the end of the reduced-diameter portion 4 in a +X direction and the end of the reduced-diameter portion 4 in a -X direction. The cover member 9 has a pair of respective projections 20 disposed on the end of an inner surface of the cover member 9 in the +X direction and the end of an inner surface of the cover member 9 in the -X direction, the projections 20 projecting into the inner space 10 in the cover member 9 and extending in the Y direction.

The protrusions 20 of the cover member 9 are inserted or positioned in the respective cover member guide grooves 19 of the reduced-diameter portion 4, so that the cover member 9 is held on the catheter body 2 for reciprocating movement along the axial directions of the catheter body 2 between a first position P1 in which the distal end of the injection needle 8 in a -Y direction is housed in the inner space 10 as depicted in FIG. 1 and a second position P2 in which the distal end of the injection needle 8 in the -Y direction projects forwardly, i.e., in the -Y direction as depicted in FIG. 4.

When the cover member 9 is positioned in the first position P1 depicted in FIG. 1, the operating member 17 of the diaphragm mechanism 15 that is inserted in the operating member guide groove 14 of the cover member 9 is in an angular position oriented essentially in the +X direction as depicted in FIG. 3, in which the blades 18 of the diaphragm mechanism 15 are closed, squeezing the outer circumferential portion of the tubular member 6 disposed in the opening 5 in the catheter body 2 thereby to close the lumen 7 in the tubular body 6 which is in the form of a soft tube.

When the cover member 9 moves in a +Y direction until it is positioned in the second position P2 depicted in FIG. 4 with respect to the catheter body 2, since the operating member guide groove 14 of the cover member 9 extends helically, the operating member 17 of the diaphragm mechanism 15 that is inserted in the operating member guide groove 14 of the cover member 9 is brought into an angular position oriented essentially in the -X direction, opening the blades 18 of the diaphragm mechanism 15 thereby to open the lumen 7 in the tubular body 6, as depicted in FIG. 5.

In other words, when the cover member 9 is positioned in the first position P1, in which the distal end of the injection needle 8 in the -Y direction is stored or housed in the inner space 10, the lumen 7 in the tubular body 6 is closed, and when the cover member 9 is positioned in the second position P2, in which the distal end of the injection needle 8 in the -Y direction projects forwardly from the inner space 10, the lumen 7 in the tubular body 6 is open.

In addition, the cover member 9 is resiliently pressed forwardly with respect to the catheter body 2, i.e., in the -Y direction, by the helical resilient member 13 that is disposed between the partition plate 11 in the cover member 9 and the front end of the catheter body 2. Therefore, unless an external force acts on the cover member 9, the cover member 9 is positioned in the first position P1 depicted in FIG. 1.

The catheter body 2 should preferably be made of a material which is flexible to a certain extent, such as metal or resin. The metal may be, for example, pseudoelastic alloy (including superelastic alloy) such as Ni-Ti alloy, stainless steel (for example, all SUS types such as SUS304, SUS303, SUS316, SUS316L, SUS316J1, SUS316J1L, SUS405, SUS430, SUS434, SUS444, SUS429, SUS430F, SUS302, etc.), cobalt alloy, precious metal such as gold or platinum, tungsten-base alloy, carbon-base material (including piano wire), or the like. The resin may be, for example, polyolefin (for example, polyethylene, polypropylene, polybutene, ethylenepropylene copolymer, ethylene-vinyl acetate copolymer, ionomer, a mixture of two or more of these materials, or the like), a polymeric material such as polyvinyl chloride, polyamide, polyamide elastomer, polyester, polyester elastomer, polyurethane, polyurethane elastomer, polyimide, fluororesin, or the like, or a mixture of these materials, or two or more of the above polymeric materials. The resin may also be engineering plastics typified by polyetheretherketone. The catheter body 2 may also include a multilayer tube made of a composite material including any of the metal and resin materials referred to above.

The cover member 9 may also be made of the similar material as the catheter body 2.

The tubular body 6 is may be made of a pliable material such as silicone rubber or the like, for example.

The operating member 17 and the blades 18 of the diaphragm mechanism 15 may be made of a sufficiently rigid material, such as a metal material or a resin material, for example.

FIG. 6 depicts the makeup of a medical injection system that uses the medication injecting catheter 1 according to Embodiment 1. A medication supply 23 and a pressure gage 22 are connected to the proximal end of the medication injecting catheter 1 through a connector 21, and a controller 24 is connected to the pressure gage 22 and the medication supply 23.

The pressure gage 22 detects the pressure in the medication supply lumen 3 of the medication injecting catheter 1. The medication supply 23, which includes a syringe pump, for example, supplies a medication to the medication supply lumen 3 of the medication injecting catheter 1. The controller 24 may control the supplying of the medication from the medication supply 23 so that the pressure detected by the pressure gage 22 in the medication supply lumen 3 of the medication injecting catheter 1 may be constant.

The medical injection system thus arranged is capable of holding the pressure in the medication supply lumen 3 at a constant level.

An example of operation of the medication injecting catheter 1 according to Embodiment 1 will be described below.

First, as depicted in FIG. 7, a guiding catheter G is inserted into the body of a patient, e.g., into the heart, and the medication injecting catheter 1 is inserted into the guiding catheter 1 until the distal end of the medication injecting catheter 1 projects out of the guiding catheter G.

The medication supply lumen 3 of the medication injecting catheter 1 has been filled with a medication by the medication supply 23. When the cover 9 is not held in contact with an inner wall W of the heart, since no external forces act on the cover member 9, the cover 9 is positioned in the first position P1 so that the distal end of the injection needle is housed inside the cover member 9. As the distal end of the injection needle 8 is housed in the inner space 10 and the lumen 7 in the tubular body 6 is closed, no medication leaks out of the injection needle 8.

The medication injecting catheter 1 is advanced until the distal end of the cover member 9 is brought into contact with the inner wall W of the heart. When the medication injecting catheter 1 pressed against the inner wall W of the heart, as depicted in FIG. 8, the cover member 9 is moved into the second position P2. The distal end of the injection needle 8 projects forwardly from the inner space 10 and pierces the inner wall W of the heart, and the lumen 7 in the tubular body 6 is opened. The pressure in the medication supply lumen 3 is lowered, and based on a detection signal from the pressure gage 22 that has detected the pressure drop, the controller 24 actuates the medication supply 23 to start supplying the medication to the medication supply lumen 3. The medication in the medication supply lumen 3 is injected into the heart tissue through the lumen 7 in the tubular body 6 and the injection needle 8.

When the inner wall W of the heart moves away from the medication injecting catheter 1 and leaves the distal end of the cover member 9 as the heart pulsates, the injection needle 8 is pulled out of the inner wall W of the heart, and at the same time the cover member 9 moves from the second position P2 to the first position P1, whereupon the lumen 7 in the tubular body 6 is closed. Therefore, the medication stops being injected from the injection needle 8.

When the inner wall W of the heart moves again toward the medication injecting catheter 1 and the cover member 9 moves from the first position P1 to the second position P2 as the heart pulsates, the distal end of the injection needle 8 projects forwardly from the inner space 10 and pierces the inner wall W of the heart, whereupon the lumen 7 in the tubular body 6 is opened. The medication is now once again injected into the heart tissue.

In this manner, the injection of the medication into the heart tissue and the ceasing of the injection of the medication are repeated as the heart pulsates.

When the cover member 9 is positioned in the first position P1, having the distal end of the injection needle 8 stored in the inner space 10, the diaphragm mechanism 15 closes the lumen 7 in the tubular body 6, and when the cover member 9 is positioned in the second position P2, having the distal end of the injection needle 8 projecting forwardly from the inner space 10, the diaphragm mechanism 15 opens the lumen 7 in the tubular body 6. Therefore, the injection needle 8 automatically pierces the inner wall W of the heart in synchronism with the pulsation of the heart for reliably injecting the medication into the heart tissue without confirming whether the injection needle has pierced the inner wall W of the heart or not. When the injection needle 8 is pulled out of the inner wall W of the heart, the injection of the medication from the injection needle 8 is automatically stopped to prevent the medication from leaking out. Consequently, an appropriate amount of medication can reliably be administered, and the medication is prevented from being unnecessarily injected into regions other than the target region.

Because the injection of the medication and the ceasing of the injection of the medication are automatically carried out in response to the movement of the cover member 9 with respect to the catheter body 2, the injecting procedure is simplified.

The tissue into which the mediation is injected is not limited to heart tissue. Though the medication injecting catheter is able to inject the medication automatically into a heart by utilizing the pulsation of the heart, the medication injecting catheter may not necessarily require a tissue to pulsate for injecting the medication into the tissue. It is possible for the medication injecting catheter to inject the medication into other organs by pressing the distal end of the catheter against the body wall and to stop injecting the medication by releasing the distal end of the catheter from the body wall, so that the injecting procedure is simplified with safety.

### Embodiment 2

In Embodiment 1, the opening and closing mechanism for opening and closing the lumen 7 in the tubular body 6 includes the diaphragm or diaphragm mechanism 15. However, the opening and closing mechanism is not limited to the diaphragm mechanism 15.

FIG. 9 depicts the makeup of a medication injecting catheter 31 according to Embodiment 2. The medication injecting catheter 31 includes an elongate catheter body 32 and a cover member 9 reciprocally movably disposed on the distal end of the catheter body 32.

In the catheter body 32, a disk-shaped rotor 33 is disposed in the recess 5 in the reduced-diameter portion 4, in place of the diaphragm mechanism 15 in the catheter body 2 used in the medication injecting catheter 1 according to Embodiment 1. The rotor 33 serves as an opening and closing mechanism and is fixed to an outer circumferential surface of an end (an end in the -Y direction) of the tubular body 6. The tubular body 6 has an end rotatably coupled or connected to the medication supply lumen 3 and another end (an end in the +Y direction) fixed to the medication supply lumen 3.

The rotor 33 is disposed in the recess 5 in the reduced-diameter portion 4 for rotation about the central axis of the catheter body 32. As depicted in FIG. 10, an operating member 34 that extends radially outwardly is mounted on a side of the rotor 33. The operating member 34 has a distal end inserted or positioned in the operating member guide groove 14 defined in the inner wall surface of the cover member 9, so that the angular position of the operating member 34 varies depending on the relative position of the cover member 9 in the Y direction with respect to the catheter body 32, causing the tubular body 6 to twist upon rotation of the rotor 33.

When the cover member 9 is positioned in the first position P1 depicted in FIG. 9, the operating member 34 of the rotor 33 that is inserted in the operating member guide groove 14 of the cover member 9 is in an angular position oriented essentially in the +X direction as depicted in FIG. 10, in which the tubular body 6 in the form of a soft tube is twisted thereby to close the lumen 7 in the tubular body 6.

When the cover member 9 moves in the +Y direction with respect to the catheter body 32 until it is positioned in the second position P2 depicted in FIG. 11, since the operating member guide groove 14 of the cover member 9 extends helically, the operating member 34 of the rotor 33 that is inserted in the operating member guide groove 14 of the cover member 9 is brought into an angular position oriented essentially in the -X direction, untwisting the tubular body 6 thereby to open the lumen 7 in the tubular body 6, as depicted in FIG. 12.

According to Embodiment 2, therefore, when the cover member 9 is positioned in the first position P1, having the distal end of the injection needle 8 in the -Y direction stored in the inner space 10, the lumen 7 in the tubular body 6 is closed, and when the cover member 9 is positioned in the second position P2, having the distal end of the injection needle 8 in the -Y direction projecting forwardly from the inner space 10, the lumen 7 in the tubular body 6 is opened. Therefore, as with Embodiment 1, the injecting procedure is simplified, and at the same time the medication can automatically and reliably be injected into the heart tissue in synchronism with the pulsation of the heart, and the medication is prevented from leaking from the injection needle 8 when the injection needle 8 is pulled out of the inner wall W of the heart.

### Embodiment 3

In Embodiment 2, the single rotor 33 fixed to one end of the tubular body 6 serves as the opening and closing mechanism. However, respective rotors may be fixed to both ends of the tubular body 6, and may twist the tubular body 6 from both of its ends for closing the lumen 7 in the tubular body 6.

FIG. 13 depicts the makeup of a medication injecting catheter 41 according to Embodiment 3. The medication injecting catheter 41 includes an elongate catheter body 42 and a cover member 43 reciprocally movably disposed on the distal end of the catheter body 42.

The catheter body 42 has rotors 33A and 33B disposed in a pair of recesses 5A and 5B, respectively, that are defined in the reduced-diameter portion 4 at a spaced interval in the Y direction, in place of the single rotor 33 disposed in the recess 5 in the reduced-diameter portion 4 of the catheter body 32 used in the medication injecting catheter 31 according to Embodiment 2. The pair of rotors 33A and 33B serve as an opening and closing mechanism and are fixed to respective outer circumferential surfaces of both ends of the tubular body 6. The two ends of the tubular body 6 are rotatably coupled or connected to the medication supply lumen 3.

The rotors 33A and 33B each have the similar structure as the rotor 33 according to Embodiment 2, and have respective operating members 34A and 34B extending radially outwardly from sides thereof.

Of the pair of recesses 5A and 5B, the recess 5A is defined in the +Z direction that extends from the central axis of the catheter body 42, and the other recess 5B is defined in the -Z direction that extends perpendicularly from the central axis of the catheter body 42. The recesses 5A and 5B are held in fluid communication with each other through a through hole 44 defined in the catheter body 42 on its central axis. The tubular body 6 extends in the through hole 44 and have both ends positioned in the respective recesses 5A and 5B and coupled or connected to the corresponding rotors 33A and 33B.

The cover member 43 has a pair of operating member guide grooves 14A and 14B defined in an inner wall surface thereof. The operating member guide groove 14A is disposed only in an inner wall surface of the cover member 43 that lies in the +Z direction, of the entire inner wall surface of the cover member 43, in agreement with the direction along which the recess 5A is defined in the catheter body 42. The operating member guide groove 14A extends helically around the central axis of the cover member 43 along the Y direction. The other operating member guide groove 14B is disposed only in an inner wall surface of the cover member 43 that lies in the -Z direction, of the entire inner wall surface of the cover member 43, in agreement with the direction along which the recess 5B is defined in the catheter body 42. The operating member guide groove 14B extends helically around the central axis of the cover member 43 along the Y direction, wound in a twisting direction opposite the operating member guide groove 14A.

The operating member 34A that extends radially outwardly from the side of the rotor 33A has a distal end inserted in the operating member guide groove 14A of the cover member 43, whereas the operating member 34B that extends radially outwardly from the side of the rotor 33B has a distal end inserted in the operating member guide groove 14B of the cover member 43. Consequently, the angular positions of the respective operating members 34A and 34B vary depending on the relative position of the cover member 43 with respect to the catheter body 42 in the Y direction. As the rotors 33A and 33B are rotated in opposite directions, the tubular body 6 is twisted from both ends of the tubular body 6.

When the cover member 43 is positioned in the first position P1 depicted in FIG. 13, the operating member 34A of the rotor 33A that is inserted in the operating member guide groove 14A of the cover member 43 is in an angular position oriented essentially in the +Z direction, and the operating member 34B of the rotor 33B that is inserted in the operating member guide groove 14B of the cover member 43 is in an angular position oriented essentially in the -Z direction, as depicted in FIG. 13. At this time, the tubular body 6 which in the form of a soft tube is twisted from both ends of the tubular body 6 in the through hole 44 thereby closing the lumen 7 in the tubular body 6.

When the cover member 43 moves in the +Y direction until it is positioned in the second position P2 depicted in FIG. 14 with respect to the catheter body 42, the operating member 34A of the rotor 33A that is inserted in the operating member guide groove 14A of the cover member 43 and the operating member 34B of the rotor 33B that is inserted in the operating member guide groove 14B of the cover member 43 are brought into respective angular positions both oriented essentially in the -X direction, untwisting the tubular body 6 thereby to open the lumen 7 in the tubular body 6.

According to Embodiment 3, therefore, when the cover member 43 is positioned in the first position P1, in which the distal end of the injection needle 8 in the -Y direction is stored or housed in the inner space 10, the lumen 7 in the tubular body 6 is closed, and when the cover member 43 is positioned in the second position P2, in which the distal end of the injection needle 8 in the -Y direction projects forwardly or in the distal direction from the inner space 10, the lumen 7 in the tubular body 6 is opened. Therefore, as with Embodiments 1 and 2, the injecting procedure is simplified, and at the same time the medication can automatically and reliably be injected into the heart tissue in synchronism with the pulsation of the heart, and the medication is prevented from leaking from the injection needle 8 when the injection needle 8 is pulled out of the inner wall W of the heart.

According to Embodiment 3, furthermore, because the pair of rotors 33A and 33B rotate in mutually opposite directions upon movement of the cover member 43 with respect to the catheter body 42, when the rotors 33A and 33B rotate through an angle that is 1/2 of the angle through which the rotor 33 according to Embodiment 2 rotates, the tubular body 6 is twisted through the same angle. In other words, the distance between the first position P1 and the second position P2 in the Y direction may be smaller, making it possible to reduce the size of the cover member 43.

### Embodiment 4

In Embodiment 1 described above, the medication injecting catheter may further include a trap mechanism for trapping (drawing-in or capturing) the inner wall W of the heart into the front end portion of the cover member 9 by evacuating the inner space 10 in the cover member 9.

As depicted in FIG. 15, the trap mechanism includes a suction hole 51 defined in the partition plate 11 of the cover member 9 and a suction unit 52 connected to the suction hole 51. For example, the suction unit 52 may be connected to the connector 21 of the medication injecting system depicted in FIG. 6, the catheter body 2 of the medication injecting catheter 1 may have a suction lumen defined therein independently of the medication supply lumen 3, and the suction unit 52 may be connected to the suction hole 51 in the cover member 9 through the suction lumen and the connector 21.

When the suction unit 52 draws air from the suction lumen to evacuate the inner space 10 in the cover member 9, the inner wall W of the heart is trapped into or held in the front end portion of the cover member 9. It is thus possible to inject the medication more reliably into the heart tissue.

The trap mechanism may similarly be incorporated into the medical injecting catheter 31 according to Embodiment 2 and the medical injecting catheter 41 according to Embodiment 3.

### Embodiment 5

In Embodiment 1 described above, contrast markers 61 and 62 may be provided on the outer circumferential surface of the reduced-diameter of the catheter body 2 and the outer circumferential surface of the cover member 9, respectively. An example of such contrast markers 61, 62 is shown in FIG. 6. These contrast markers 61 and 62 serve to assist in grasping the positions of the reduced-diameter of the catheter body 2 and the cover member 9 contrast X-rays when the medication injecting catheter 1 is inserted into the body of a patient. The contrast markers 61 and 62 may be made of a material easily recognizable by contrast X-rays, e.g., gold, platinum, iridium, tungsten, or an alloy of any of them, or a silver-palladium alloy, or the like.

By identifying the position of the reduced-diameter of the catheter body 2 and the position of the cover member 9 using the contrast markers 61 and 62, it is possible to decide on an X-ray image whether the cover member 9 is positioned in the first position P1 as depicted in FIG. 16 or positioned in the second position P2 as depicted in FIG. 17.

Contrast markers may similarly be provided on both the reduced-diameter portion of the catheter body and the cover member of the medication injecting catheter 31 according to Embodiment 2, the medication injecting catheter 41 according to Embodiment 3, and the medication injecting catheter according to Embodiment 4.

In Embodiments 1 through 5, the injection needle 8 pierces the inner wall W of the heart and injects the medication into the inner wall W. However, the injection needle 8 may similarly pierce the outer wall of the heart and inject the medication into the outer wall. The object to be treated is not limited to the heart, but the medication injecting catheter according to the present invention is applicable where a medication is to be injected into living bodies through various body walls thereof, such as inner and outer walls of other organs, etc.

The medication may be any optional therapeutic substances. The therapeutic effects may include cardiac wall strengthening, scaffolding, proangiogenesis, cell replenishment, and tissue repair or regeneration by way of apoptosis and necrosis prevention. The therapeutic substances include a biocompatible mono- or multicomponent material that can be injected, beads on a polymer base, and polymer hydrogels. Other therapeutic substances include a fibrin adhesive, collagen, alginate, a synthetic polymeric material such as polyethylene glycol or the like, and chitosan, etc. Though the therapeutic substances may include the above materials only, they are not limited thereto. For example, they may include, individually or in any optional combination, stem cells such as induced pluripotent stem (iPS) cells or the like, fibroblast, cells such as skeletal cells or the like, protein, plasmid, genes, growth factors, chemoattractant, synthetic polypeptide, various pharmaceutical compositions, and other therapeutically useful substances.

### Description of Reference Symbols

1, 31, 41 Medication injecting catheter, 2, 32, 42 Catheter body, 3 Medication supply lumen, 4 Reduced-diameter portion, 5, 5A, 5B Recess, 6 Tubular body, 7 Lumen, 8 Injection needle, 9, 43 Cover member, 10 Inner space, 11 Partition plate, 12 Through hole, 13 Resilient member, 14, 14A, 14B Operating member guide groove, 15 Diaphragm mechanism, 16 Frame, 17, 34, 34A, 34B Operating member, 18 Blade, 19 Cover member guide groove, 20 Projection, 21 Connector, 22 Pressure gage, 23 Medication supply, 24 Controller, 44 Through hole, 51 Suction hole, 52 Suction unit, 61, 62 Contrast marker, P1 First position, P2 Second position, G Guiding catheter, W Inner wall of a heart.

## Claims

1. A medication injecting catheter (1, 31, 41) for injecting a medication into a living body through a body wall thereof, the medication injecting catheter (1, 31, 41) comprising:
an elongate catheter body (2, 32, 42) including a medication supply lumen (3) defined therein;
a tubular body (6) connected to the distal end of the catheter body (2, 32, 42) and having a lumen (7) defined therein which is held in fluid communication with the medication supply lumen (3);
an injection needle (8) connected to a distal end of the tubular body (6);
a hollow cylindrical cover member (9, 43) that includes an inner space (10) defined therein for housing at least the tubular body (6) therein, the cover member (9, 43) being disposed on a distal end portion of the catheter body (2, 32, 42) for reciprocating movement along an axial direction of the catheter body (2, 32, 42) between a first position (P1) in which a distal end of the injection needle (8) is housed in the inner space (10) and a second position (P2) in which the distal end of the injection needle (8) projects forwardly from the inner space (10);
an opening and closing mechanism that includes an operating member (17, 34, 34A, 34B) rotatable in circumferential directions of the catheter body (2, 32, 42) in the inner space (10) in the cover member (9, 43), for opening and closing the lumen (7) in the tubular body (6) depending on an angular position of the operating member (17, 34, 34A, 34B);
a motion converting mechanism for converting the reciprocating movement of the cover member (9, 43) relative to the catheter body (2, 32, 42) into rotary movement of the operating member (17, 34, 34A, 34B) to cause the opening and closing mechanism to close the lumen (7) in the tubular body (6) when the cover member (9, 43) is positioned in the first position (P1) and to cause the opening and closing mechanism to open the lumen (7) in the tubular body (6) when the cover member (9, 43) is positioned in the second position (P2); and
a resilient member (13) which is disposed between the catheter body (2, 32, 42) and the cover member (9, 43) and which resiliently presses the cover member (9, 43) forwardly of the catheter body (2, 32, 42) toward the first position (P1).

2. The medication injecting catheter (1, 31, 41) according to claim 1, wherein
the tubular member is pliable; and
the opening and closing mechanism includes a diaphragm mechanism (15) for changing an opening area of the lumen (7) in the tubular body (6) depending on the angular position of the operating member (17, 34, 34A, 34B) .

3. The medication injecting catheter (1, 31, 41) according to claim 1, wherein
the tubular member is pliable; and
the opening and closing mechanism includes at least one rotor for twisting the tubular body (6) depending on the angular position of the operating member (17, 34, 34A, 34B) .

4. The medication injecting catheter (1, 31, 41) according to any one of claims 1 through 3, wherein
the motion converting mechanism includes an operating member guide groove (14, 14A, 14B) defined helically in an inner circumferential surface of the cover member (9, 43) around a central axis of the cover member (9, 43); and
the operating member (17, 34, 34A, 34B) includes a projection (20) extending radially of the catheter body (2, 32, 42) in the inner space (10) in the cover member (9, 43) and movably inserted in the operating member guide groove (14, 14A, 14B).

5. The medication injecting catheter (1, 31, 41) according to any one of claims 1 through 4, wherein
the cover member (9, 43) includes a partition plate (11) fixedly disposed in the inner space (10) of the cover member (9, 43) perpendicular to the axial direction, the partition plate (11) including a through hole (12, 44) through which the injection needle (8) extends; and
the resilient member (13) is disposed between a front end of the catheter body (2, 32, 42) and the partition plate (11) of the cover member(9, 43).

6. The medication injecting catheter (1, 31, 41) according to any one of claims 1 through 5, further comprising:
a trap mechanism for trapping a portion of the wall of the living body in a front end portion of the cover member (9, 43) by evacuating the inner space (10) in the cover member (9, 43).

7. The medication injecting catheter (1, 31, 41) according to any one of claims 1 through 6, wherein the catheter body (2, 32, 42) and the cover member (9, 43) each include a respective contrast marker (61, 62).

8. A medication injecting system comprising:
the medication injecting catheter (1, 31, 41) according to any one of claims 1 through 7;
a pressure gage (22) that detects a pressure in the medication supply lumen (3) of the medication injecting catheter (1, 31, 41);
a medication supply (23) that supplies a medication to the medication supply lumen (3) of the medication injecting catheter (1, 31, 41); and
a controller (24) that controls supply of the medication from the medication supply (23) to the medication supply lumen (3) so that the pressure detected by the pressure gage (22) in the medication supply lumen (3) of the medication injecting catheter (1, 31, 41) is constant.

## Patentansprüche

1. Medikamenteninjektionskatheter (1, 31, 41) zum Injizieren eines Medikaments in einen lebenden Körper durch eine Körperwand desselben, wobei der Medikamenteninjektionskatheter (1, 31, 41) umfasst:
einen länglichen Katheterkörper (2, 32, 42) mit einem darin definierten Medikamentenzuführungslumen (3);
einen röhrenförmigen Körper (6), der mit dem distalen Ende des Katheterkörpers (2, 32, 42) verbunden ist und ein darin definiertes Lumen (7) aufweist, das in Fluidverbindung mit dem Medikamentenzuführungslumen (3) gehalten wird;
eine Injektionsnadel (8), die mit einem distalen Ende des rohrförmigen Körpers (6) verbunden ist;
ein hohles zylindrisches Abdeckelement (9, 43), das einen darin definierten Innenraum (10) zur Aufnahme zumindest des röhrenförmigen Körpers (6) enthält, wobei das Abdeckelement (9, 43) an einem distalen Endabschnitt des Katheterkörpers (2, 32, 42) zur Hin- und Herbewegung entlang einer axialen Richtung des Katheterkörpers (2, 32, 42) zwischen einer ersten Position (P1), in der ein distales Ende der Injektionsnadel (8) in dem Innenraum (10) untergebracht ist, und einer zweiten Position (P2), in der das distale Ende der Injektionsnadel (8) nach vorne aus dem Innenraum (10) herausragt, angeordnet ist;
einen Öffnungs- und Schließmechanismus, der ein Bedienelement (17, 34, 34A, 34B) umfasst, das in Umfangsrichtungen des Katheterkörpers (2, 32, 42) in dem Innenraum (10) in dem Abdeckelement (9, 43) drehbar ist, um das Lumen (7) in dem rohrförmigen Körper (6) in Abhängigkeit von einer Winkelposition des Bedienelements (17, 34, 34A, 34B) zu öffnen und zu schließen;
einen Bewegungsumwandlungsmechanismus zum Umwandeln der Hin- und Herbewegung des Abdeckelements (9, 43) relativ zu dem Katheterkörper (2, 32, 42) in eine Drehbewegung des Bedienelements (17, 34, 34A, 34B), um zu bewirken, dass der Öffnungs- und Schließmechanismus das Lumen (7) in dem röhrenförmigen Körper (6) schließt, wenn das Abdeckelement (9, 43) in der ersten Position (P1) positioniert ist, und zu bewirken, dass der Öffnungs- und Schließmechanismus das Lumen (7) in dem rohrförmigen Körper (6) öffnet, wenn das Abdeckelement (9, 43) in der zweiten Position (P2) positioniert ist; und
ein elastisches Element (13), das zwischen dem Katheterkörper (2, 32, 42) und dem Abdeckelement (9, 43) angeordnet ist und das das Abdeckelement (9, 43) elastisch vor den Katheterkörper (2, 32, 42) in Richtung der ersten Position (P1) drückt.

2. Medikamenteninjektionskatheter (1, 31, 41) nach Anspruch 1, wobei
das röhrenförmige Element biegsam ist; und
der Öffnungs- und Schließmechanismus einen Diaphragma-Mechanismus (15) zum Ändern einer Öffnungsfläche des Lumens (7) in dem röhrenförmigen Körper (6) in Abhängigkeit von der Winkelposition des Bedienelements (17, 34, 34A, 34B) umfasst.

3. Medikamenteninjektionskatheter (1, 31, 41) nach Anspruch 1, wobei
das rohrförmige Element biegsam ist; und
der Öffnungs- und Schließmechanismus mindestens einen Rotor zum Verdrehen des röhrenförmigen Körpers (6) in Abhängigkeit von der Winkelposition des Bedienelements (17, 34, 34A, 34B) umfasst.

4. Medikamenteninjektionskatheter (1, 31, 41) nach einem der Ansprüche 1 bis 3, wobei
der Bewegungsumwandlungsmechanismus eine Bedienelement-Führungsnut (14, 14A, 14B) enthält, die schraubenförmig in einer inneren Umfangsfläche des Abdeckelements (9, 43) um eine Mittelachse des Abdeckelements (9, 43) herum definiert ist; und
das Bedienelement (17, 34, 34A, 34B) einen Vorsprung (20) aufweist, der sich radial vom Katheterkörper (2, 32, 42) in den Innenraum (10) im Abdeckelement (9, 43) erstreckt und beweglich in die Führungsnut (14, 14A, 14B) des Bedienelements eingesetzt ist.

5. Medikamenteninjektionskatheter (1, 31, 41) nach einem der Ansprüche 1 bis 4, wobei
das Abdeckelement (9, 43) eine Trennplatte (11) aufweist, die fest in dem Innenraum (10) des Abdeckelements (9, 43) senkrecht zur axialen Richtung angeordnet ist, wobei die Trennplatte (11) ein Durchgangsloch (12, 44) aufweist, durch das sich die Injektionsnadel (8) erstreckt; und
das elastische Element (13) zwischen einem vorderen Ende des Katheterkörpers (2, 32, 42) und der Trennplatte (11) des Abdeckelements (9, 43) angeordnet ist.

6. Medikamenteninjektionskatheter (1, 31, 41) nach einem der Ansprüche 1 bis 5, ferner umfassend:
einen Einfangmechanismus zum Einfangen eines Teils der Wand des lebenden Körpers in einem vorderen Endabschnitt des Abdeckelements (9, 43) durch Evakuieren des Innenraums (10) in dem Abdeckelement (9, 43).

7. Medikamenteninjektionskatheter (1, 31, 41) nach einem der Ansprüche 1 bis 6, wobei der Katheterkörper (2, 32, 42) und das Abdeckelement (9, 43) jeweils eine Kontrastmarkierung (61, 62) aufweisen.

8. System zur Injektion von Medikamenten, umfassend:
den Medikamenteninjektionskatheter (1, 31, 41) nach einem der Ansprüche 1 bis 7;
ein Druckmessgerät (22), das einen Druck im Medikamentenzuführungslumen (3) des Medikamenteninjektionskatheters (1, 31, 41) erfasst;
eine Medikamentenzuführung (23), die ein Medikament in das Medikamentenzuführungslumen (3) des Medikamenteninjektionskatheters (1, 31, 41) zuführt; und
eine Steuerung (24), die die Zufuhr des Medikaments von der Medikamentenzuführung (23) zu dem Medikamentenzuführungslumen (3) so steuert, dass der von dem Druckmesser (22) in dem Medikamentenzuführungslumen (3) des Medikamenteninjektionskatheters (1, 31, 41) erfasste Druck konstant ist.

## Revendications

1. Un cathéter d'injection de médicament (1, 31, 41) pour l'injection d'un médicament dans un corps vivant à travers une paroi corporelle de celui-ci, le cathéter d'injection de médicament (1, 31, 41) comprenant :
un corps de cathéter allongé (2, 32, 42) comprenant une lumière d'alimentation en médicament (3) définie à l'intérieur ;
un corps tubulaire (6) connecté à l'extrémité distale du corps de cathéter (2, 32, 42) et comportant une lumière (7) qui est maintenue en communication fluide avec la lumière d'alimentation en médicament (3) ;
une aiguille d'injection (8) connectée à une extrémité distale du corps tubulaire (6) ;
un élément de couvercle cylindrique creux (9, 43) qui comprend un espace intérieur (10) défini à l'intérieur pour y loger au moins le corps tubulaire (6), l'élément de couvercle (9, 43) étant disposé sur une partie d'extrémité distale du corps de cathéter (2, 32, 42) pour un mouvement réciproque le long d'une direction axiale du corps de cathéter (2, 32, 42) entre une première position (P1) dans laquelle une extrémité distale de l'aiguille d'injection (8) est logée dans l'espace intérieur (10) et une seconde position (P2) dans laquelle l'extrémité distale de l'aiguille d'injection (8) fait saillie vers l'avant à partir de l'espace intérieur (10) ;
un mécanisme d'ouverture et de fermeture qui comprend un organe de commande (17, 34, 34A, 34B) tournable dans les directions circonférentielles du corps de cathéter (2, 32, 42) dans l'espace intérieur (10) dans l'élément de couvercle (9, 43), pour ouvrir et fermer la lumière (7) dans le corps tubulaire (6) en fonction d'une position angulaire de l'organe de commande (17, 34, 34A, 34B) ;
un mécanisme de conversion de mouvement pour convertir le mouvement réciproque de l'élément de couvercle (9, 43) par rapport au corps de cathéter (2, 32, 42) en un mouvement rotatif de l'organe de commande (17, 34, 34A, 34B) pour causer le mécanisme d'ouverture et de fermeture à fermer la lumière (7) dans le corps tubulaire (6) lorsque l'élément de couvercle (9, 43) est positionné dans la première position (P1) et pour causer le mécanisme d'ouverture et de fermeture à ouvrir la lumière (7) dans le corps tubulaire (6) lorsque l'élément de couvercle (9, 43) est positionné dans la seconde position (P2) ; et
un élément élastique (13) qui est disposé entre le corps de cathéter (2, 32, 42) et l'élément de couvercle (9, 43) et qui presse de manière élastique l'élément de couvercle (9, 43) vers l'avant du corps de cathéter (2, 32, 42) en direction de la première position (P1).

2. Le cathéter d'injection de médicament (1, 31, 41) selon la revendication 1, dans lequel
l'élément tubulaire est pliable ; et
le mécanisme d'ouverture et de fermeture comprend un mécanisme à diaphragme (15) pour modifier une aire d'ouverture de la lumière (7) dans le corps tubulaire (6) en fonction de la position angulaire de l'organe de commande (17, 34, 34A, 34B).

3. Le cathéter d'injection de médicament (1, 31, 41) selon la revendication 1, dans lequel
l'élément tubulaire est pliable ; et
le mécanisme d'ouverture et de fermeture comprend au moins un rotor pour tordre le corps tubulaire (6) en fonction de la position angulaire de l'organe de commande (17, 34, 34A, 34B).

4. Le cathéter d'injection de médicament (1, 31, 41) selon l'une quelconque des revendications 1 à 3, dans lequel
le mécanisme de conversion de mouvement comprend une rainure de guidage de l'organe de commande (14, 14A, 14B) définie de manière hélicoïdale dans une surface circonférentielle intérieure de l'élément de couvercle (9, 43) autour d'un axe central de l'élément couvercle (9, 43) ; et
l'organe de commande (17, 34, 34A, 34B) comprend une saillie (20) s'étendant radialement du corps de cathéter (2, 32, 42) dans l'espace intérieur (10) de l'élément de couvercle (9, 43) et étant insérée de manière déplaçable dans la rainure de guidage de l'organe de commande (14, 14A, 14B).

5. Le cathéter d'injection de médicament (1, 31, 41) selon l'une quelconque des revendications 1 à 4, dans lequel
l'élément couvercle (9, 43) comprend une plaque de séparation (11) disposée de manière fixe dans l'espace intérieur (10) de l'élément de couvercle (9, 43) perpendiculairement à la direction axiale, la plaque de séparation (11) comprenant un trou de passage (12, 44) à travers lequel s'étend l'aiguille d'injection (8) ; et
l'élément élastique (13) est disposé entre une extrémité avant du corps de cathéter (2, 32, 42) et la plaque de séparation (11) de l'élément de couvercle (9, 43).

6. Le cathéter d'injection de médicament (1, 31, 41) selon l'une quelconque des revendications 1 à 5, comprenant en outre :
un mécanisme de piégeage pour une partie de la paroi du corps vivant dans une partie d'extrémité avant de l'élément de couvercle (9, 43) en évacuant l'espace intérieur (10) dans l'élément de couvercle (9, 43).

7. Le cathéter d'injection de médicament (1, 31, 41) selon l'une quelconque des revendications 1 à 6, dans lequel le corps de cathéter (2, 32, 42) et l'élément de couvercle (9, 43) comprennent chacun un marqueur de contraste respectif (61, 62).

8. Un système d'injection de médicament comprenant :
le cathéter d'injection de médicament (1, 31, 41) selon l'une quelconque des revendications 1 à 7 ;
un manomètre (22) qui détecte une pression dans la lumière d'alimentation en médicament (3) du cathéter d'injection de médicament (1, 31, 41) ;
une unité d'alimentation en médicaments (23) qui fournit un médicament à la lumière d'alimentation en médicament (3) du cathéter d'injection de médicament (1, 31, 41) ; et
un contrôleur (24) qui contrôle l'alimentation du médicament de l'unité d'alimentation en médicament (23) à la lumière d'alimentation en médicament (3) de manière à ce que la pression détectée par le manomètre (22) dans la lumière d'alimentation en médicament (3) du cathéter d'injection de médicament (1, 31, 41) soit constante.
